# FASCICULE DE BREVET EUROPEEN

(11) **EP 0 618 001 B1**
(45) Date de publication et mention de la délivrance du brevet: **13.08.1997**
(21) Numéro de dépôt: 94400650.1
(22) Date de dépôt: 28.03.1994
(51) Int. Cl.: B01J 2/06, A23K 1/16

(54) **Procédé de préparation de sphérules de principes actifs**
Verfahren zur Herstellung von Wirkstoffe enthaltenden Kügelchen
Process for preparing active agents containing spherules

(30) Priorité: 31.03.1993 FR 9303728
(43) Date de publication de la demande: 05.10.1994
(73) Titulaire: RHONE-POULENC NUTRITION ANIMALE, 92160 Antony (FR)
(72) Inventeur: Dollat, Jean-Marie, F-03100 Montlucon (FR); Molin, Marc, F-03100 Néris les Bains (FR); Theallier, Pascal, F-03100 Montlucon (FR)
(74) Mandataire: Le Pennec, Magali

(56) Documents cités:
- EP-A- 0 285 682
- EP-A- 0 528 201
- WO-A-84/02255
- GB-A- 1 198 513
- DATABASE WPI Derwent Publications Ltd., London, GB; AN 79-30313B & JP-A-54 031 085 (OTA SEIYAKU) 7 Mars 1979

## Description

La présente invention concerne un nouveau procédé de stabilisation de principes actifs alimentaires et/ou médicamenteux. Elle concerne aussi un procédé de préparation de sphérules de principes actifs présentant une dispersion très réduite.

Parmi les principes actifs alimentaires et/ou médicamenteux utilisables pour préparer les sphérules selon l'invention, on peut citer parmi les vitamines : la vitamine A, la vitamine E, la vitamine B₁₂, la vitamine H ou biotine, la vitamine D₃, la vitamine PP, le vitamine K₃ ou menadione, la vitamine B₁ ou thiamine, la vitamine B₂ ou riboflavine, la vitamine B₃ ou niacine, la vitamine B₅ ou acide panthoténique, la vitamine B₆ ou pyridoxine ; parmi les caroténoïdes : le β carotène, l'asthaxanthine, la canthaxanthine ; parmi les enzymes : la β glucanase, la xylanase.

On préfère, parmi ces vitamines stabiliser les vitamines A et E.

Les vitamines A et E sont largement utilisées en alimentation animale pour favoriser la croissance des animaux. Les aliments pour animaux étant souvent préparés par un procédé qui consiste à former des particules par l'action conjointe de la pression et de la chaleur, les principes actifs sensibles à la chaleur et à la pression, tel que la vitamine A, ne peuvent subir cette procédure sans subir d'importantes dégradations.

Afin de préserver, par exemple la vitamine A, il est connu depuis longtemps, de la protéger en la mélangeant avec des protéines réticulées en présence d'un aldéhyde.

Divers procédés de réticulation des protéines en présence ou en l'absence de la vitamine A ont été décrits. La réticulation des protéines en présence de vitamine A ou E pose un problème supplémentaire par rapport à la réticulation en l'absence de vitamine A ou E. La vitamine A sous forme acétate ou la vitamine E sont des produits huileux qui ne se mélangent avec les protéines et leur agent de réticulation que sous la forme d'une émulsion huile dans eau qui n'est jamais facile à manipuler. La réticulation de la protéine exige en plus un chauffage pendant une période relativement longue, ce qui n'est pas favorable à la stabilité de ces vitamines.

Un premier procédé de réticulation de la gélatine en présence d'acétaldéhyde pour protéger la vitamine A est par exemple proposé dans le brevet EP 261 616. Dans ce brevet, on réalise un mélange "intime" contenant la protéine, un alcool miscible à l'eau, l'acétaldéhyde et environ 5% d'eau et la vitamine A, dans ce mélange, dit "intime", la vitamine A se présente sous forme de gouttes de moins de 10 microns. Ce mélange est lyophilisé de façon à obtenir des particules solides de diamètre compris entre 100 et 800 microns. Les particules solides sont ensuite soumises à des vapeurs d'acétaldéhyde pendant une période d'environ 3 heures à une température comprise entre 50 et 60°C.

Cette méthode de préparation ne peut être réalisée selon un procédé continu car elle est réalisée en deux étapes, chacune d'entre elles exigeant un type d'appareil différent, un lyophiliseur et un appareil de pulvérisation. La lyophilisation est parmi ces deux étapes, l'étape la plus couteuse car elle a toujours une productivité extrêmement limitée ce qui rend le produit obtenu très onéreux.

Un deuxième procédé de préparation de sphérules de vitamine A est décrit dans le brevet EP 285 682. Selon ce procédé on prépare une émulsion contenant la vitamine, de l'eau, de la gélatine et un sucre que l'on transforme en gouttes par pulvérisation, ces gouttes sont mises en contact individuellement avec une poudre de cellulose qui doit présenter des caractéristiques bien particulières, cette mise en contact de chacune des gouttes avec la poudre de cellulose est réalisée selon différentes techniques jusqu'à durcissement des gouttes. Ensuite les gouttes durcies sont séparées de la poudre de cellulose par tamisage, le tamis retenant les gouttes durcies et laissant passer la poudre ce qui entraine un choix stricte de la dimension granulométrique de la poudre de cellulose et surtout une difficulté sur sa nature à ne pas s'agglomérer durant la mise en oeuvre du procédé. Les gouttes durcies sont ensuite séchées puis soumises à une opération de chauffage afin d'assurer la réticulation de la gélatine par réaction des groupes aminés de la gélatine avec les fonctions carboxyliques du sucre. Ce procédé est particulièrement difficile à mettre en oeuvre car il exige un choix strict des matières utilisées et une surveillance particulièrement étroites des conditions de mise en oeuvre du procédé.

La présente invention a permis de résoudre les problèmes laissés par l'ensemble de l'art antérieur et a surtout permis d'atteindre un procédé très facile à mettre en oeuvre.

Elle consiste, dans une première étape, à former des sphérules par division contrôlée, notamment par passage dans un mélangeur:
. d'un premier mélange à base d'une émulsion primaire huile dans l'eau constituée d'une charge alimentaire ou médicamenteuse, d'une protéine, d'eau et d'huile,
. et d'un solvant non miscible à l'eau puis à séparer les sphérules obtenues.

Les principes actifs alimentaires ou médicamenteux sont de préférence choisis parmi les vitamines sous forme huileuse choisies par exemple parmi la vitamine A ou la vitamine E ou parmi des principes actifs dissous ou dispersés dans une huile alimentaire. Les huiles alimentaires sont choisies par exemple parmi les huiles végétales ou animales telles que l'huile d'arachide, de tournesol, de colza, de foie de morue.

La solution huileuse peut éventuellement contenir un ou plusieurs agent antioxydant tel que le tertiobutyl-3 hydroxy-4 anisole (BHA), le ditertiobutyl-3,5,hydroxy-4 toluène (BHT), l'éthoxy-6 dihydroxy-1,2 triméthyl-2,2,4 quinoléine (éthoxyquine), le tertiobutyl-2 dihydroxy-1,4 benzène (vendu sous la marque EMBANOX), ainsi que la vitamine E. Cette solution peut aussi contenir un ou plusieurs agents tensioactifs choisis notamment parmi le thiodipropionate de dilauryle vendu sous la marque Irganox, un stéarate alcalin ou alcalinoterreux, le stearoyl-2 lactylate de sodium ou de calcium, la carboxyméthylcellulose.

Selon une meilleure manière de mettre en oeuvre l'invention pour la formation de sphérules de vitamine A, la solution huileuse de vitamine a la composition pondérale suivante:

| | |
|---|---|
| - acétate de vitamine A | 70 à 80 % |
| - agent antioxydant | 10 à 30 % |
| - tensioactif | 0 à 5 % |

La protéine est mise en solution dans l'eau. On préfère utiliser la gélatine. Cette solution peut aussi éventuellement contenir un agent tensioactif, choisi notamment parmi les tensioactifs précédemment cités.

La solution aqueuse de la protéine est de préférence une solution aqueuse contenant environ 10 à 60 % (de préférence 20 à 30 %) en poids de gélatine et contenant éventuellement environ 10 à 60 % (de préférence 10 - 20 %) en poids de sucre (glucose, lactose, fructose, saccharose, maltodextrine) ou de glycérol.

L'émulsion primaire est formée par dispersion de la solution huileuse contenant les principes actifs alimentaires et/ou médicamenteux dans la solution aqueuse contenant la protéine à une température supérieure au point de gélification de la solution.

L'émulsion primaire est de préférence réalisée par mélange d'environ 10 à 30% en poids de solution huileuse et de 70 à 90 % en poids de solution aqueuse de la protéine. D'autres mélanges, contenant des quantités différentes de chacune des solutions, sont bien sûrs réalisables, ils ne sortent pas du cadre de l'invention, les quantités préconisées n'étant que préférentielles.

Le solvant non miscible à l'eau utilisé pour former les sphérules est choisi de préférence parmi les solvants aliphatiques contenant 4 à 8 atomes de carbone. On préfère particulièrement utiliser une coupe aliphatique contenant 6 atomes de carbone. Il peut aussi contenir un agent tensioactif tel qu'un ester de saccharose (0,1 à 1%).

Après formation des sphérules, on peut utiliser un agent de réticulation de la protéine qui est choisi, par exemple, parmi l'acétaldéhyde, le glutaraldéhyde, le glyoxal. L'agent de réticulation éventuellement utilisé peut être mis en oeuvre à l'état pur ou en solution aqueuse selon une concentration comprise entre 5 et 20 %.

Les différents procédés de mise en oeuvre de l'invention vont maintenant être décrits.

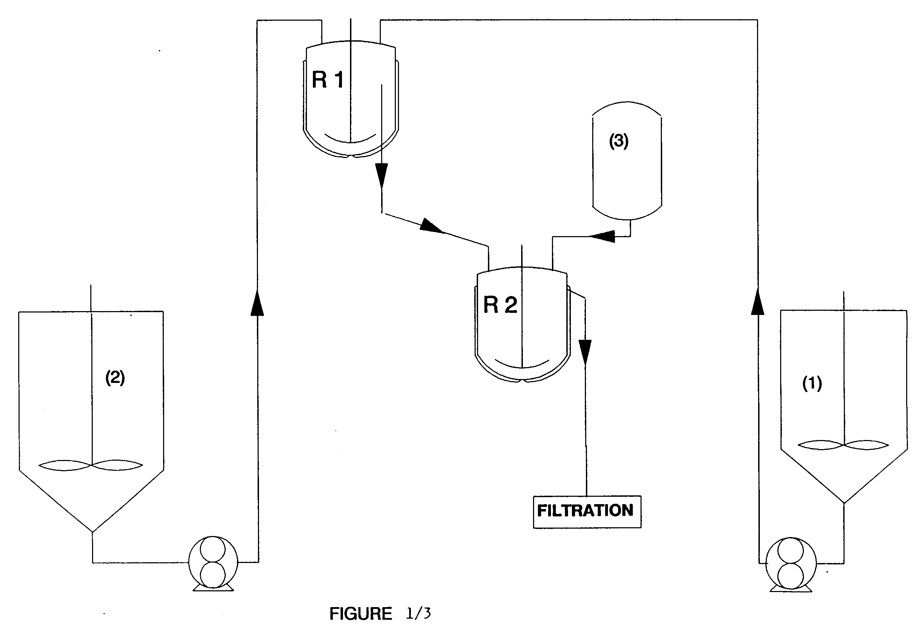

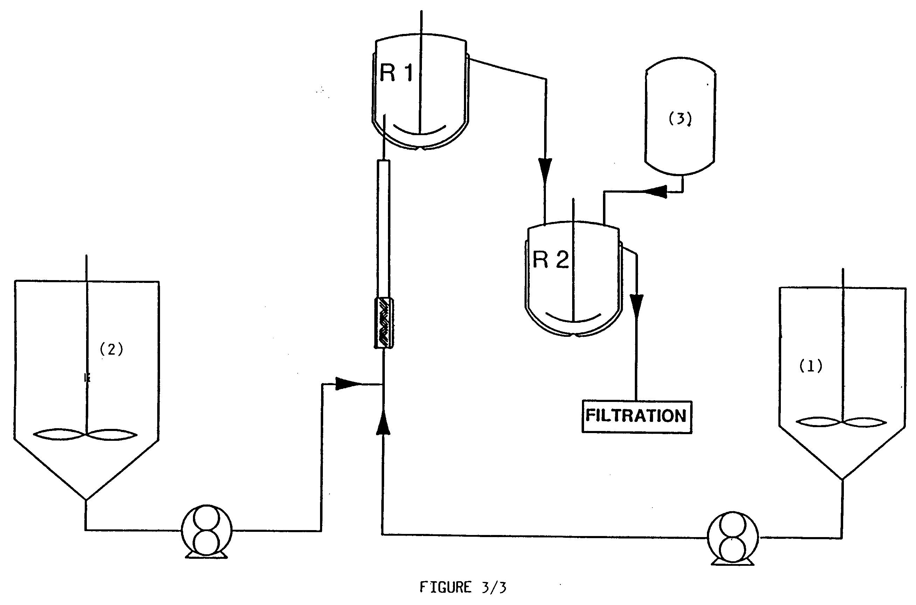

Selon un premier procédé de mise en oeuvre représenté sur la figure 1/3, la formation des sphérules est réalisée par introduction dans un mélangeur (R₁) muni d'une turbine tournant à une vitesse suffisante pour réaliser la dispersion souhaitée de l'émulsion primaire (1) dans le solvant non miscible à l'eau (2) contenant éventuellement un émulsifiant. Ce réacteur est maintenu de préférence à une température comprise entre 35°C et 70°C, et encore plus préférentiellement entre 45°C et 55°C.

Par débordement du réacteur de granulation ou par tout autre moyen, on introduit les sphérules formées précédemment dans un réacteur de refroidissement-réticulation (R₂) maintenu à environ 20°C et agité par une turbine de façon à éviter la coalescence des sphérules, dans ce réacteur on introduit éventuellement l'agent de réticulation (3).

La formation des sphérules et leur réticulation peut être réalisée selon un procédé continu par exemple à l'aide d'une série de réacteurs en cascade dont le remplissage se fait par débordement du réacteur en amont vers le réacteur en aval ou par un procédé discontinu. Il est bien évident qu'un procédé continu présente des avantages économiques notables.

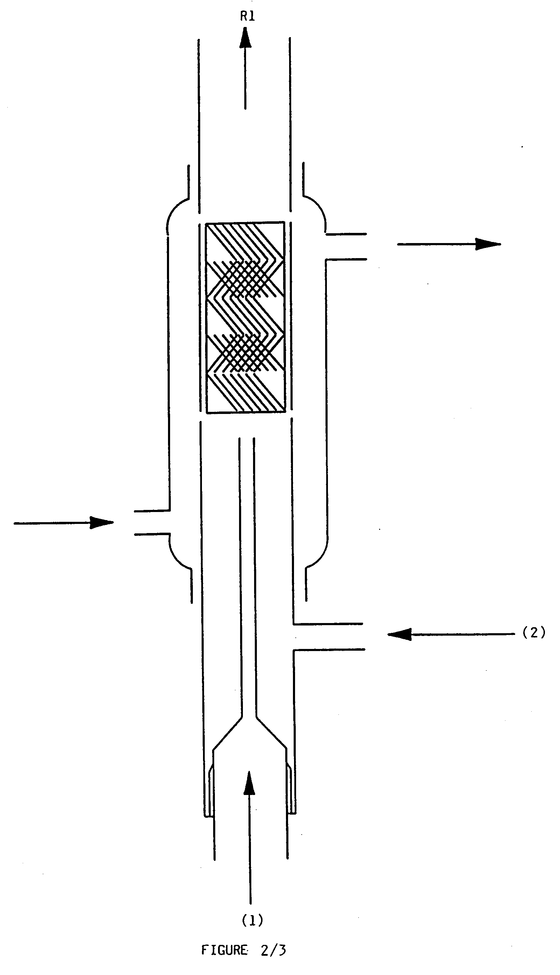

Selon un deuxième procédé de mise en oeuvre, la formation des sphérules est réalisée selon la figure 2/3 par passage dans un mélangeur statique de la première émulsion (1) et du solvant (2). On adaptera la vitesse et la température de la première émulsion et du solvant à la dimension des sphérules que l'on souhaite obtenir. Le rapport des phases volumiques émulsion primaire sur solvant est compris entre 0,2 et 1.

Le mélangeur statique est constitué d'un tube contenant des pièces métalliques entrecroisées. Parmi les types de mélangeur statique utilisables en peut citer ceux vendus sous la marque SULZER tel que décrit dans le brevet français publié sous le numéro 2 468 401. Ce type d'appareillage est utilisé pour réaliser des mélanges homogènes de différents fluides et des mélanges intimes de produits visqueux dans un produit de moindre viscosité. Ce type d'appareil n'a jamais été utilisé à ce jour pour former des sphérules ayant un diamètre de 100 µm à 500 µm à partir d'une émulsion primaire visqueuse ayant un diamètre moyen de l'ordre de 1 µm et d'un solvant non miscible. Il est tout à fait étonnant qu'un mélangeur statique utilisé jusqu'à ce jour pour obtenir des mélanges intimes puisse permettre d'obtenir des sphérules ayant un diamètre moyen fixé entre 100 µm et 500 µm et présentant une faible dispersion.

Les sphérules à la sortie du mélangeur statique (voir figure 3/3) sont refroidies jusqu'à environ la température ambiante sous agitation dans une réacteur R₁ de façon à éviter leur coalescence. On ajoute ensuite éventuellement l'agent de réticulation (3) de la protéine dans un réacteur R2 puis on sépare les sphérules par filtration.

L'ensemble des procédés décrits permet une production continue de sphérules de principes actifs alimentaires et/ou médicamenteux dans des appareils de petites tailles et n'exige pas d'étape de séchage avant de procéder à la réticulation et n'exige pas, non plus, la présence de matière pulvérente différente de la composition recherchée . Il présente donc un énorme avantage par rapport à l'ensemble des procédés connus à ce jour.

La présente invention sera plus complètement décrite à l'aide des exemples suivants qui ne doivent pas être considérés comme limitatifs de l'invention.

### EXEMPLE

### SOLUTION AQUEUSE DE LA PROTEINE

Dans un réacteur de 50 litres agité avec une turbine disperseuse tournant à 450-500 tours/minute, on introduit:
- 14,2 litres d'eau
- 1 kg de glycérine

On chauffe à 60°C. Sous agitation, on ajoute :
- 3,7 kg de lactose
- 6,2 kg de gélatine

On continue à agiter pendant 35 minutes à 60°C.

### SOLUTION DE LA CHARGE ALIMENTAIRE

Dans un réacteur, on mélange la charge vitaminée constituée de:
- 2,92 kg de vitamine A sous forme acétate
- 0,75 kg de BHT
- 0,23 kg d'Irganox®

On chauffe l'ensemble à environ 60°C et on le verse dans le réacteur précédent dans lequel la turbine est portée à 3000 tours/minute. On continue à agiter pendant 20 minutes en maintenant à 60°C. On obtient une émulsion dont le diamètre, des particules est d'environ un micron.

### PREPARATION DU SOLVANT

On dissout 1,6 g d'ester de saccharose dans 1 litre d'isohexane.

### 1er PROCEDE DE MISE EN OEUVRE DE L'INVENTION

### APPAREILLAGE (FIGURE 1/3)

Le réacteur de granulation (R₁) est un réacteur en verre de 0,5 l possédant une double enveloppe et équipé d'une turbine RAYNERI tournant à une vitesse de 800 à 1000 tours/minute.

Le réacteur de refroidissement-réticulation (R₂) est un réacteur en verre, muni d'une double enveloppe de 0,5 l équipé d'une turbine RAYNERI tournant à 500 tours/minute.

L'émulsion est stockée dans un réacteur de 5 litres maintenu à 60°C.

Le solvant est stocké dans un réacteur de 10 litres maintenu à 50°C.

On introduit dans le réacteur de granulation (R₁) 250 ml de solvant puis on alimente l'émulsion préparée précédemment à un débit de 1,7 litre par heure.

Après 5 minutes d'agitation, on alimente le solvant à un débit de 3,4 litres par heure. Quand le volume atteint 350 ml dans R₁, le réacteur déborde dans le réacteur (R₂) de refroidissement-réticulation qui est un réacteur de 0,5 litre en verre contenant 400 ml de solvant. La température dans R₂ est régulée à 20°C par circulation d'eau froide dans la double enveloppe. Dès que le réacteur (R₂) déborde par le trop plein, on introduit en continu dans (R₂) une solution aqueuse de glutaraldéhyde (16,7 % en poids) à un débit de 50 ml/heure.

Les particules réticulées ainsi obtenues sont isolées par filtration puis séchées en lit fluidisé. La granulométrie des particules présente les caractéristiques suivantes:
Diamètre moyen: 320 µm
Indice de dispersion: 0,344
Sphérules supérieures à 500 µm = 4,3%
Sphérules inférieures à 100 µm = 4,9%

On entend par indice de dispersion le résultat de la différence entre le diamètre où 84% des particules passent et le diamètre où 16 % des particules passent, divisé par deux fois le diamètre moyen.

### 2ème PROCEDE DE MISE EN OEUVRE DE L'INVENTION

### APPAREILLAGE (Figures 2/3 et 3/3)

L'émulsion est introduite (figure 3/3) dans un récipient (1) sous agitation et le solvant dans un un autre récipient (2).

L'émulsion est indroduite dans le mélangeur statique (figure 2/3) en (1) à un débit d'environ 1,2 litre par heure et le solvant en (2) à un débit de 4 litres par heure. Le mélangeur est chauffé à une température d'environ 50°C par circulation d'eau chaude dans une double enveloppe.

Le mélangeur se présente sous la forme d'une colonne garnie de pièces métalliques, ayant un diamètre de 4 mm et une hauteur d'environ 4 cm.

A la sortie du mélangeur statique (figure 3/3), l'émulsion est entrainée vers un réacteur (R₁) de refroidissement où une agitation est maintenue de façon à éviter la coalescence des particules jusqu'à ce que la température atteigne environ 20°C.

L'émulsion est ensuite entraînée vers un réacteur (R₂) ou une solution aqueuse de l'agent de réticulation à 17 % est ajoutée en continu à raison de 0,68 g de glutaraldéhyde pour 100 g d'émulsion.

Les particules réticulées sont séparées par filtration puis séchées en lit fluidisé.

La granulométrie des particules présente les caractéristiques suivantes:
Diamètre moyen = 315 µm
Indice de dispersion = 0,30
Particules supérieures à 500 µm = 1,1 %
Particules inférieures à 100 µm = 1,2 %

## Revendications

1. Procédé de préparation de sphérules d'un ou de plusieurs principes actifs alimentaires ou médicamenteux caractérisé en ce que dans une première étape on forme des sphérules par division contrôlée d'une émulsion primaire d'huile dans l'eau constituée d'une charge alimentaire ou médicamenteuse, d'huile, d'une protéine et d'eau dans un solvant non miscible à l'eau puis dans une deuxième étape on sépare les sphérules obtenues.

2. Procédé selon la revendication 1 caractérisé en ce que la charge alimentaire contient des vitamines choisies parmi les vitamines A, E, D₃, B₁₂, H, K₃, PP, B₁, B₂, B₃, B₅, B₆.

3. Procédé selon la revendication 1 caractérisé en ce que la protéine est la gélatine.

4. Procédé selon la revendication 1 caractérisé en ce que l'huile est choisie parmi la vitamine A, la vitamine E, l'huile de colza, l'huile d'arachide, de tournesol ou de foie de morue.

5. Procédé selon la revendication 1 caractérisé en ce que l'émulsion contient en plus un agent antioxydant choisi parmi le tertiobutyl-3 hydroxy-4 anisole, le ditertiobutyl-3,5 hydroxy-4 toluène, l'éthoxy-6 dihydroxy-1,2 triméthyl-2,2,4 quinoléine et le tertiobutyl-2 dihydroxy-1,4 toluène.

6. Procédé selon la revendication 1 caractérisé en ce que l'émulsion contient en plus un agent tensioactif choisi parmi le thiodipropionate de dilauryle, un stéarate alcalin ou alcalinoterreux, le stéaroyl-2 lactylate de sodium ou de calcium, ou la carboxyméthylcellulose.

7. Procédé selon la revendication 1 caractérisé en ce que le solvant est choisi parmi les solvants aliphatiques contenant 4 à 8 atomes de carbone.

8. Procédé selon l'une quelconque des revendications 1 à 6 caractérisé en ce que l'émulsion primaire est formée par mélange d'une solution huileuse à base ou contenant un ou plusieurs principes actifs et éventuellement un ou plusieurs agents antioxydants et d'une solution aqueuse contenant la protéine, et éventuellement un ou plusieurs agents tensioactifs.

9. Procédé selon la revendication 8 caractérisé en ce que l'émulsion primaire est formée par un mélange d'environ 10 à 30 % en poids de la solution huileuse et de 70 à 90 % en poids de la solution aqueuse.

10. Procédé selon l'une quelconque des revendications précédentes caractérisé en ce qu'avant de procéder à la séparation des sphérules on effectue une étape de réticulation.

11. Procédé selon la revendication 10 caractérisé en ce que l'agent de réticulation est choisi parmi l'acétaldéhyde, le glutaraldéhyde, le glyoxal.

12. Procédé selon la revendication 11 caractérisé en ce que l'agent de réticulation est constitué par une solution aqueuse contenant 5 à 20 % en poids d'agent de réticulation ou le produit pur.

13. Procédé de préparation de sphérules présentant un diamètre compris entre 100 µm et 500 µm selon l'une quelconque des revendications précédentes caractérisé en ce qu'elles sont formées par introduction dans un mélangeur statique ou dans un mélangeur agité de l'émulsion contenant le ou les principes actifs, la protéine et l'eau et d'un solvant non miscible à l'eau.

## Claims

1. Process for the preparation of spherules of one or more alimentary or medicinally active principles, characterized in that in a first step spherules are formed by controlled division of a primary oil-in-water emulsion consisting of an alimentary or medicinal charge, oil, a protein and water in a water-immiscible solvent, and in a second step the spherules obtained are then separated.

2. Process according to claim 1, characterized in that the alimentary charge contains vitamins chosen from vitamins A, E, D₃, B₁₂, H, K₃, PP, B₁, B₂, B₃, B₅, B₆.

3. Process according to claim 1, characterized in that the protein is gelatin.

4. Process according to claim 1, characterized in that the oil is chosen from vitamin A, vitamin E or rapeseed, groundnut, sunflower or cod liver oil.

5. Process according to claim 1, characterized in that the emulsion additionally contains an antioxidant agent chosen from 3-tert-butyl-4-hydroxyanisole, 3,5-di-tert-butyl-4-hydroxytoluene, 6-ethoxy-1,2-dihydroxy-2,2,4-trimethylquinoline and 2-tert-butyl-1,4-dihydroxytoluene.

6. Process according to claim 1, characterized in that the emulsion additionally contains a surface-active agent chosen from dilauryl thiodipropionate, an alkali-metal or alkaline-earth metal stearate, sodium or calcium 2-stearoyllactate or carboxymethylcellulose.

7. Process according to claim 1, characterized in that the solvent is chosen from aliphatic solvents containing 4 to 8 carbon atoms.

8. Process according to any one of claims 1 to 6, characterized in that the primary emulsion is formed by mixing an oily solution based on or containing one or more active principles and optionally one or more antioxidizing agents and an aqueous solution containing the protein, and optionally one or more surface-active agents.

9. Process according to claim 8, characterized in that the primary emulsion is formed by mixing from approximately 10 to 30 % by weight of the oily solution and from 70 to 90 % by weight of the aqueous solution.

10. Process according to any one of the preceding claims, characterized in that before separation of the spherules is performed a crosslinking step is carried out.

11. Process according to claim 10, characterized in that the crosslinking agent is chosen from acetaldehyde, glutaraldehyde and glyoxal.

12. Process according to claim 11, characterized in that the crosslinking agent consists of an aqueous solution containing 5 to 20 % by weight of crosslinking agent or the pure product.

13. Process for the preparation of spherules having a diameter between 100 µm to 500 µm according to any one of the preceding claims, characterized in that they are formed by introduction into a static mixer or into a stirred mixer of the emulsion containing the active principle(s), the protein, water and a water-immiscible solvent.

## Patentansprüche

1. Verfahren zur Herstellung von Kügelchen von einem oder mehreren medikamentösen Wirkstoffen oder Nahrungsmittel-Wirkstoffen, dadurch gekennzeichnet, daß man in einer ersten Stufe Kügelchen durch kontrollierte Verteilung einer primären Emulsion von Öl-in-Wasser bildet, bestehend aus einem Nahrungsmittel-Füllstoff oder medikamentösen Füllstoff, Öl, einem Protein und Wasser in einem mit Wasser nicht mischbaren Lösungsmittel, und anschließend in einer zweiten Stufe die erhaltenen Kügelchen abtrennt.

2. Verfahren nach Anspruch 1, dadurch gekennzeichnet, daß der Nahrungsmittel-Füllstoff Vitamine enthält, ausgewählt unter den Vitaminen A, E, D₃, B₁₂, H, K₃, PP, B₁, B₂, B₃, B₅, B₆.

3. Verfahren nach Anspruch 1, dadurch gekennzeichnet, daß das Protein Gelatine ist.

4. Verfahren nach Anspruch 1, dadurch gekennzeichnet, daß das Öl unter Vitamin A, Vitamin E, Rapsöl, Erdnußöl, Sonnenblumenöl oder Lebertran ausgewählt wird.

5. Verfahren nach Anspruch 1, dadurch gekennzeichnet, daß die Emulsion außerdem ein Antioxidationsmittel enthält, ausgewählt unter 3-tert.-Butyl-4-hydroxy-anisol, 3,5-ditert.-Butyl-4-hydroxy-toluol, 6-Ethoxy-1,2-dihydroxy-2,2,4-trimethyl-chinolin und 2-tert.-Butyl-1,4-dihydroxy-toluol.

6. Verfahren nach Anspruch 1, dadurch gekennzeichnet, daß die Emulsion außerdem ein oberflächenaktives Mittel enthält, ausgewählt unter Thiodipropionsäure-dilaurylester, einem Alkalistearat oder Erdalkalistearat, Natrium-2-stearoyl-lactylat, Calcium-2-stearoyl-lactylat oder Carboxymethylcellulose.

7. Verfahren nach Anspruch 1, dadurch gekennzeichnet, daß das Lösungsmittel unter den aliphatischen Lösungsmitteln mit 4 bis 8 Kohlenstoffatomen ausgewählt wird.

8. Verfahren nach irgendeinem der Ansprüche 1 bis 6, dadurch gekennzeichnet, daß die primäre Emulsion durch Mischen einer öligen Lösung auf der Basis von oder enthaltend ein(em) oder mehrer(n) Wirkstoff(en) und gegebenenfalls ein(em) oder mehrere(n) Antioxidationsmittel(n) und einer wässrigen Lösung gebildet wird, die das Protein und gegebenenfalls ein oder mehrere oberflächenaktive Mittel enthält.

9. Verfahren nach Anspruch 8, dadurch gekennzeichnet, daß die primäre Emulsion durch Mischen von etwa 10 bis 30 Gew.-% der öligen Lösung und 70 bis 90 Gew.-% der wäßrigen Lösung gebildet wird.

10. Verfahren nach irgendeinem der vorstehenden Ansprüche, dadurch gekennzeichnet, daß man vor der Abtrennung der Kügelchen eine Stufe der Vernetzung durchführt.

11. Verfahren nach Anspruch 10, dadurch gekennzeichnet, daß das Vernetzungsmittel unter Acetaldehyd, Glutaraldehyd und Glyoxal ausgewählt wird.

12. Verfahren nach Anspruch 11, dadurch gekennzeichnet, daß das Vernetzungsmittel aus einer wäßrigen Lösung besteht, die 5 bis 20 Gew.-% Vernetzungsmittel oder das reine Produkt enthält.

13. Verfahren zur Herstellung von Kügelchen mit einem Durchmesser zwischen 100 µm und 500 µm nach irgendeinem der vorstehenden Ansprüche, dadurch gekennzeichnet, daß sie durch Eintragen der Emulsion, die den oder die Wirkstoffe, das Protein und Wasser und ein mit Wasser nicht mischbares Lösungsmittel enthält, in einen statischen Mischer oder einen gerührten Mischer gebildet wird.
